# EUROPEAN PATENT APPLICATION

(11) **EP 3 815 641 A1**
(43) Date of publication of application: **05.05.2021**
(21) Application number: 19206883.1
(22) Date of filing: 04.11.2019
(51) Int. Cl.: A61B 18/02, A61B 5/00, A61B 34/20, A61B 18/14, A61B 18/00

(54) **ELECTROYPHYSIOLOGICAL GUIDANCE AND VISUALIZATION FOR BALLOON, AND METHODS THERAPY AND ASSOCIATED DEVICES, SYSTEMS**

(71) Applicant: Koninklijke Philips N.V., 5656 AG Eindhoven (NL)
(72) Inventor: IBRAGIMOV, Zalman, 5656 AE Eindhoven (NL); SCHWARTZ, Yitzhack, 5656 AE Eindhoven (NL)
(74) Representative: Philips Intellectual Property & Standards

(57) **Abstract**

Devices, systems, and methods for guiding a balloon therapy procedure by generating and outputting a visualization of the volumetric position of a balloon within an anatomical cavity are provided. For example, in one embodiment, a processor circuit is configured to control a plurality of electrodes to detect an electromagnetic field within the anatomical cavity, and determine, based on the detected electromagnetic field and a geometrical parameter associated with the balloon (e.g., size, shape of the balloon), a location of the balloon within the anatomical cavity. The processor circuit outputs a signal representation of a visualization of the balloon to guide placement of the therapeutic balloon. In some embodiments, the visualization is output to a display with a map of the anatomical cavity. The visualization may be updated based on detected movement of the balloon to provide a real time view of the location of the balloon within the anatomical cavity.

## Description

### TECHNICAL FIELD

The present disclosure relates generally to electrophysiological imaging for guiding treatment procedures, and, in particular, to electrophysiological systems and methods for imaging volumes of the body and guiding balloon therapy procedures. For example, a mapping and guidance system can include a processing circuit configured to control an electrophysiology catheter to guide a balloon to be positioned at a treatment site.

### BACKGROUND

Therapeutic balloons have been used recently to treat a variety of different ailments and disorders, including blood vessel occlusions and heart arrhythmias. For example, atrial fibrillation (AF) is an abnormal heart rhythm characterized by rapid and irregular beating of the atria, and may be associated with heart palpitations, fainting, lightheadedness, shortness of breath, or chest pain. The disease is associated with an increased risk of heart failure, dementia, and stroke. AF may be caused by electrical pulses generated by secondary pacers at the ostium of the pulmonary veins. Accordingly, one way of treating AF is by pulmonary vein isolation, which can include ablating the inner wall of the left atrium to form lesions that isolate the ostium of the pulmonary veins from the rest of the left atrium. Ablation can be performed in various ways, including radiofrequency (RF) ablation, ultrasonic ablation, and cryoablation. RF ablation is a conventional ablation procedure that involves powering an RF electrode to create contiguous, transmural lesions using heat energy. RF ablation suffers from some drawbacks, such as a longer procedure time and small gaps in the lesions that cause AF to return over time, and even immediately.

Balloon-based therapy procedures, such as balloon angioplasty and balloon ablation can provide several advantages, including a shorter procedure time and the ability to treat an entire circumferential area (e.g., a blood vessel, pulmonary vein ostium) with a single deployment. For example, in cryoablation, an inflatable cryoballoon is inflated and cooled to a temperature (e.g., below -65° C) that causes an electrically-isolating lesion or firewall in the tissue. In conventional systems, the cryoballoon is maneuvered to and positioned at the ablation site, typically the ostium of a pulmonary vein. The cryoballoon is inflated, cooled, and positioned to fully occlude blood flow of the pulmonary vein from the left atrium. In this way, it can be assured that the lesion formed from the cryoablation will electrically isolate the pulmonary vein from the left atrium. As another example, a RF balloon comprising a plurality of electrodes can be maneuvered to and positioned at the ablation site, typically the ostium of a pulmonary vein. The RF balloon is positioned such that it fully occludes blood flow of the pulmonary vein from the left atrium. In this way, it can be assured that the lesion formed from the energy delivered by the electrodes electrically isolates the pulmonary vein from the left atrium.

Some challenges with balloon therapy include guiding the ablation balloon (e.g., cryoballoon) to the ablation site, and ensuring that the balloon is placed and oriented to maintain contact with a full circumference of the ostium. If the balloon is misaligned during ablation, the resulting lesion can include one or more gaps which result in reconduction and recurrence of the arrhythmia thereby requiring a re-do procedure when the AF symptoms return.

In conventional methods, angiographic and/or fluoroscopic procedures are used to guide the balloon to the ablation site. Once the inflated balloon is in place, a contrast agent or dye is introduced into the pulmonary vein to detect leaks or gaps in the interface between the balloon and the ostium of the pulmonary vein prior to ablating the tissue. When gaps exist, a portion of contrast agent will leak through into the left atrium. This leaked portion can be detected under fluoroscopy, which indicates that the balloon is not yet fully occluding blood flow from the pulmonary vein into the left atrium, and therefore is not optimally positioned to completely isolate the pulmonary vein from the left atrium. Accordingly, the physician can adjust the balloon until no residual leaks of the contrast agent are seen.

There are some shortcomings to using angiography and contrast agent to guide balloon ablation procedures. For example, patients and physicians may prefer to avoid x-ray radiation emitted during angiography and fluoroscopy procedures. Furthermore, guiding the balloon to the ablation site may be an imprecise and difficult processes that requires special expertise. It may be unadvisable to use contrast agent for some patients, and the contrast agent may not adequately identify leaks between the ostium of the pulmonary vein and the balloon. For example, at least 20% of the population has some type of contraindication for contrast agents, including allergic reactions and kidney failure.

### SUMMARY

Aspects of the present disclosure provide devices, systems, and methods for guiding a balloon therapy procedure by providing a visualization of the volumetric position of the balloon within an anatomical cavity of a patient. For example, in one embodiment, a processor circuit is configured to control a plurality of electrodes (e.g., EP catheter electrodes) to detect an electromagnetic (EM) field, and determine the location of the balloon based on the detected EM field and a geometric parameter associated with the balloon. In some embodiments, the geometric parameter may include an assumption or known geometric characteristic of the balloon (e.g., size, position relative to catheter), and can be used by the processor circuit as an input to a location determination function for identifying and visualizing the inflated balloon. In some embodiments, the visualization is output to a display along with a map or image of the anatomical cavity. The visualization may be updated based on detected movement of the balloon to provide a real time view of the location of the balloon within the anatomical cavity.

According to one embodiment of the present disclosure, an apparatus for guiding balloon therapy within an anatomical cavity comprises a processor circuit configured for communication with a plurality of electrodes. In some embodiments, the processor circuit is configured to: control the plurality of electrodes to detect an electromagnetic field within the anatomical cavity; receive a geometrical parameter associated with a balloon configured to provide the balloon therapy within the anatomical cavity; determine, based on the detected electromagnetic field and the geometrical parameter associated with the balloon, a location of the balloon within the anatomical cavity; and output a signal representative of a visualization of the location of the balloon within the anatomical cavity.

In some embodiments, the processor circuit is configured to: control the plurality of electrodes to detect the electromagnetic field at a first time before the balloon is inflated and a second time after the balloon is inflated, compute, based on the electromagnetic field detected at the first time, a first conductance map of the anatomical cavity corresponding to the first time; compute, based on the electromagnetic field detected at the second time, a second conductance map of the anatomical cavity corresponding to the second time; compare the first conductance map and the second conductance map to determine a change in a conductance field of the anatomical cavity between the first time and the second time; and determine, based on the geometrical parameter and the determined change in the conductance field, the location of the balloon within the anatomical cavity. In some embodiments, the processor circuit is configured to control a plurality of catheter-mounted electrodes to detect the electromagnetic field at the first time and the second time. In some embodiments, the processor circuit is configured to control the plurality of catheter-mounted electrodes to emit the electromagnetic field at the first time and the second time. In some embodiments, the processor circuit is configured to control the plurality of catheter-mounted electrodes to detect the electromagnetic field based on combinations of a receiving electrode of the plurality of catheter-mounted electrodes and an emitting frequency. In some embodiments, the processor circuit is configured to control a plurality of external electrodes to emit the electromagnetic field at the first time and the second time.

In some aspects, the processor circuit is configured to: control a plurality of catheter-mounted electrodes to detect the electromagnetic field within the anatomical cavity; control the plurality of catheter-mounted electrodes to detect a fault condition in which each catheter-mounted electrode of the plurality of catheter-mounted electrodes is occluded; and determine the location of the balloon within the anatomical cavity based on the detected fault condition and the geometric parameter associated with the balloon. In some embodiments, the geometrical parameter comprises one or more of: a relative positioning of the balloon relative to the plurality of electrodes; a shape of the balloon; a boundary condition of the balloon within the anatomical cavity; or a geometric dimension of the balloon when inflated. In some embodiments, the visualization of the location of the balloon comprises a graphical representation of a shape of the balloon when inflated, within the anatomical cavity. In some embodiments, the processor circuit is configured to update the graphical representation of the shape of the balloon positioned relative to the anatomical cavity, in real time, based on the detected electromagnetic field. In some embodiments, the processor circuit is configured to output the signal representative of a visualization of the location of the balloon to a display in communication with the processor circuit.

According to another embodiment of the present disclosure, a system for guiding balloon therapy within an anatomical cavity comprises: an apparatus as described herein; and an electrophysiology catheter comprising an elongate tip member configured to be positioned distally of the balloon. In some embodiments, the plurality of electrodes is positioned on the elongate tip member.

According to another embodiment of the present disclosure, a method for guiding balloon therapy within an anatomical cavity comprises: controlling a plurality of electrodes to detect an electromagnetic field within the anatomical cavity; receiving a geometrical parameter associated with a balloon configured to provide the balloon therapy within the anatomical cavity; determining, based on the detected electromagnetic field and the geometrical parameter associated with the balloon, a location of the balloon within the anatomical cavity; and outputting a signal representative of a visualization of the location of the balloon within the anatomical cavity.

In some embodiments, controlling the plurality of electrodes comprises: controlling the plurality of electrodes to detect the electromagnetic field at a first time before inflating the balloon; and controlling the plurality of electrodes to detect the electromagnetic field at a second time after inflating the balloon. In some embodiments, determining the location of the balloon within the anatomical cavity comprises: computing, based on the electromagnetic field detected at the first time, a first conductance map of the anatomical cavity corresponding to the first time; computing, based on the electromagnetic field detected at the second time, a second conductance map of the anatomical cavity corresponding to the second time; comparing the first conductance map and the second conductance map to determine a change in a conductance field of the anatomical cavity between the first time and the second time; and determining, based on the geometrical parameter and the determined change in the conductance field, the location of the balloon within the anatomical cavity.

In some embodiments, controlling the plurality of electrodes to detect the electromagnetic field comprises controlling a plurality of catheter-mounted electrodes to detect the electromagnetic field. In some embodiments, the method further comprises controlling the plurality of catheter-mounted electrodes to emit the electromagnetic field. In some embodiments, controlling the plurality of catheter-mounted electrodes to detect the electromagnetic field comprises controlling the plurality of catheter-mounted electrodes to detect the electromagnetic field based on combinations of a receiving electrode of the plurality of catheter-mounted electrodes and an emitting frequency.

In some embodiments, controlling the plurality of electrodes to emit the electromagnetic field comprises controlling a plurality of external electrodes to emit the electromagnetic field. In some embodiments, controlling the plurality of electrodes comprises controlling a plurality of catheter-mounted electrodes to detect the electromagnetic field within the anatomical cavity. In some embodiments, determining the location of the balloon within the anatomical cavity comprises: controlling the plurality of catheter-mounted electrodes to detect a fault condition in which each catheter-mounted electrode of the plurality of catheter-mounted electrodes is occluded; and determining, based on the detected fault condition and the geometric parameter associated with the balloon, the location of the balloon within the anatomical cavity.

In some embodiments, the geometrical parameter comprises one or more of: a relative positioning of the balloon relative to the plurality of electrodes; a shape of the balloon; a boundary condition of the balloon within the anatomical cavity; or a geometric dimension of the balloon when inflated. In some embodiments, outputting the visualization of the location of the balloon comprises outputting a graphical representation of a shape of the balloon when inflated, within the anatomical cavity. In some embodiments, the method further comprises updating the graphical representation of the shape of the balloon positioned relative to the anatomical cavity, in real time, based on the detected electromagnetic field.

In accordance with another embodiment of the present disclosure, a computer program product includes: a non-transitory computer-readable medium having program code recorded thereon, the program code including: code for controlling a plurality of electrodes to detect an electromagnetic field within an anatomical cavity; code for receiving a geometrical parameter associated with a balloon configured to provide therapy within the anatomical cavity; code for determining, based on the detected electromagnetic field and the geometrical parameter associated with the balloon, a location of the balloon within the anatomical cavity; and code for outputting a visualization of the location of the balloon within the anatomical cavity.

Additional aspects, features, and advantages of the present disclosure will become apparent from the following detailed description.

### BRIEF DESCRIPTION OF THE DRAWINGS

Illustrative embodiments of the present disclosure will be described with reference to the accompanying drawings, of which:
Fig. 1 is a graphical depiction of a cryoballoon and EP catheter positioned at the PV ostium with complete occlusion, according to aspects of the present disclosure.
Fig. 2 is a diagrammatic view of an EP-guided cryoablation system, according to aspects of the present disclosure.
Fig. 3 is a diagrammatic view of a processor circuit, according to aspects of the present disclosure.
Fig. 4 is a flow diagram of a method for providing EP-based balloon visualization and guidance for a balloon ablation procedure, according to aspects of the present disclosure.
Fig. 5 is a graphical view of a user interface for EP-guided balloon ablation, according to aspects of the present disclosure.
Fig. 6 is a graphical view of a graphical user interface for providing EP-based balloon visualization and guidance for a balloon ablation procedure.
Fig. 7 is a flow diagram of a method for providing EP-based balloon visualization and guidance for a balloon ablation procedure using conductance mapping, according to aspects of the present disclosure.
Fig. 8 is a diagrammatic view of a natives matrix of electrical signals used to determine PV occlusion by a balloon, according to aspects of the present disclosure.
Fig. 9 is a flow diagram of a method for providing EP-based balloon visualization and guidance for a balloon ablation procedure using an EP electrode fault detection technique, according to aspects of the present disclosure.
Fig. 10 is a cross-sectional view of a therapeutic balloon positioned at a stenosed segment within a vessel, according to aspects of the present disclosure.

### DETAILED DESCRIPTION

For the purposes of promoting an understanding of the principles of the present disclosure, reference will now be made to the embodiments illustrated in the drawings, and specific language will be used to describe the same. It is nevertheless understood that no limitation to the scope of the disclosure is intended. Any alterations and further modifications to the described devices, systems, and methods, and any further application of the principles of the present disclosure are fully contemplated and included within the present disclosure as would normally occur to one skilled in the art to which the disclosure relates. For example, although the following disclosure may refer to embodiments that include balloon therapy procedures, cryoablation, cyroballoons, cryocatheters, balloon angioplasty, RF balloon ablation, or RF balloons, it will be understood that such embodiments are exemplary, and are not intended to limit the scope of the disclosure to those applications. For example, it will be understood that the devices, systems, and methods described herein are applicable to a variety of treatment procedures in which a balloon is used to occlude a body lumen or body cavity. In particular, it is fully contemplated that the features, components, and/or steps described with respect to one embodiment may be combined with the features, components, and/or steps described with respect to other embodiments of the present disclosure. For the sake of brevity, however, the numerous iterations of these combinations will not be described separately.

As mentioned above, fluoroscopy-based approaches used in guiding balloon therapy procedures, including navigation and deployment of the balloon at the therapy site (e.g., blood vessel stenosis, pulmonary vein ostium), suffer from various drawbacks. It may be desirable to provide an approach for guiding a balloon treatment procedure without using fluoroscopy and/or contrast agent. The present disclosure provides systems, methods, and devices for guiding a balloon therapy procedure using electrical field detection for balloon visualization within an anatomical map. As will be further described below, an electrophysiology system can be used to provide for image-guided delivery of a balloon, such as an ablation balloon, an angioplasty balloon, or a scoring balloon into an anatomical cavity, such as the left atrium or a blood vessel, and to facilitate deployment and placement of the balloon at the treatment site.

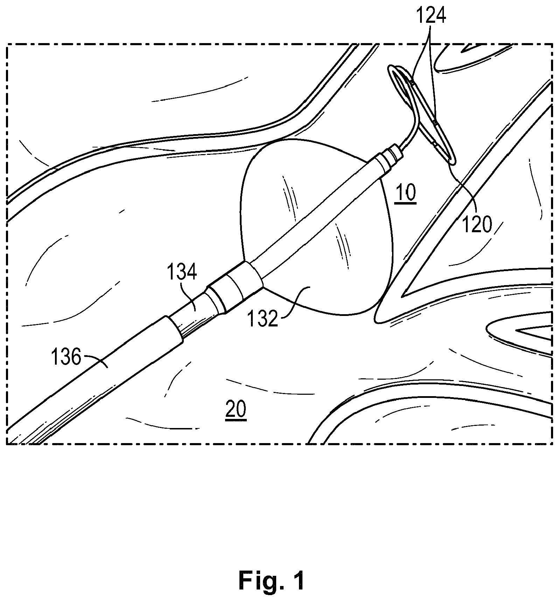

In that regard, Fig. 1 is a graphical view of an inflated ablation balloon 132 positioned at the ostium of the pulmonary vein 10. In an exemplary embodiment, the balloon 132 is cryoballoon. The cryoballoon 132 is configured to be inflated and then at least partially filled with a cooling fluid to cool the cryoballoon to a temperature (e.g., below -65° C) that causes an electrically-isolating lesion or firewall in the tissue. For example, the cryoballoon 132 may be in fluid communication with a source or reservoir of cooling fluid via one or more fluid lines positioned within a flexible elongate member 134. Further, the cryoballoon 132 may be in fluid communication with an air or gas source, e.g., for balloon inflation, via one or more fluid lines positioned within the flexible elongate member 134. The balloon 132 may be coupled to a distal portion of a flexible elongate member 134 that is protruding out a distal end of a sheath 136. In some embodiments, the sheath 136 is first introduced into the left atrium, and used to guide the EP catheter 120 to the ablation site. The balloon 132 may then be moved over the EP catheter 120 to the ablation site and positioned such that a full circumference of the balloon 132 is in contact with an entire circumference of the ostium of the pulmonary vein 10, and such that the ablation can be delivered around the entire circumference. In this way, a full occlusion is achieved, which increases the likelihood that the ablation results in complete electrical isolation. The approaches described in the present disclosure advantageously allow visualized guidance and placement of an ablation balloon at the treatment site.

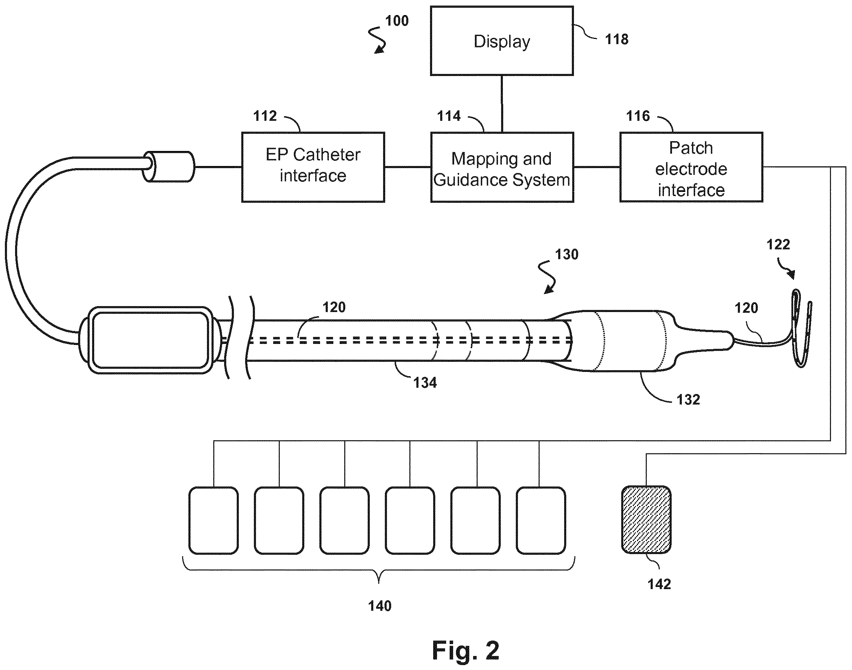

Fig. 2 is a diagrammatic schematic view of an EP cryoablation system 100, according to aspects of the present disclosure. The EP cryoablation system 100 includes an EP catheter 120. In some embodiments, the EP catheter 120 extends through a lumen of a cryoballoon catheter 130. The EP catheter 120 is communicatively coupled to an EP catheter interface 112, which is communicatively coupled to a mapping and guidance system 114. The cryoballoon catheter 130 may include an inflatable cryoballoon 132 coupled to a distal portion of a flexible elongate member 134, which may comprise a sheath. The EP catheter 120 may be positioned at least partially within a lumen of the flexible elongate member 134 such that a distal portion 122 of the EP catheter 120, which comprises a plurality of electrodes positioned on an elongate tip member, may protrude out a distal end of the cryoballoon catheter 130. For example, the cryoballoon catheter 130 may comprise a lumen configured to slidably receive the EP catheter 120. The EP catheter 120 may comprise a flexible elongate member configured to be positioned within the cryoballoon catheter 130. In some embodiments, the cryoballoon catheter 130 may be introduced into the body cavity first and the EP catheter 120 may be advanced distally within the cryoballoon catheter 130 until the distal portion 122 of the EP catheter 120 protrudes out the distal end of the cryoballoon catheter 130 at a region of interest (e.g., an ablation site).

The distal portion 122 of the EP catheter may comprise a plurality of electrodes positioned on the elongate tip member. In some embodiments, the EP catheter comprises between 8 and 10 electrodes. However, the EP catheter can include other numbers of electrodes, including 2, 4, 6, 14, 20, 30, 60, or any other suitable number of electrodes, both larger and smaller. The elongate tip member may be configured to be positioned distally of the cryoballoon, and may be biased, shaped, or otherwise structurally configured to assume a shape, such as a circular shape, in which the electrodes are spaced from one another about one or more planes. For example, the EP catheter can be a spiral mapping catheter (SMC) in which electrodes are distributed along an elongate tip member in a spiral configuration. In some embodiments, commercially-available EP catheters can be used with the system 100, including the Achieve™ and Achieve Advance™ Mapping catheters manufactured by Medtronic™. The EP catheter can be designed for use with the Arctic Front™ Family of Cardiac Cryoablation Catheters and/or the FlexCath™ Advance Steerable Sheath, manufactured by Medtronic™. In some embodiments, the cryoballoon 132 comprises a plurality of electrodes positioned on an exterior surface of the cryoballoon 132 and configured to obtain data used to determine occlusion at an ablation site. Further details regarding EP catheters and assemblies can be found in, for example, U.S. Patent No. 6,002,955, titled "Stabilized Electrophysiology Catheter and Method for Use," the entirety of which is hereby incorporated by reference.

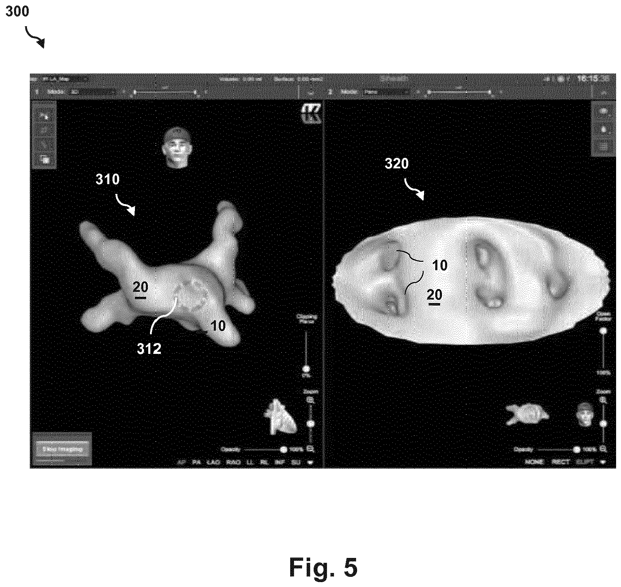

The system 100 further comprises a plurality of body patch electrodes 140 and a reference patch electrode 142 communicatively coupled to a patch electrode interface 116, which is in communication with the mapping and guidance system 114. For example, the patch electrodes 140 and the reference electrode 142 may be coupled to the patch electrode interface 116 via electrical cables. In the embodiment shown in Fig. 2, the system 100 comprises six external body patch electrodes 140 and one reference electrode 142. However, in some embodiments, the system 100 comprises fewer or more body patch electrodes 140 than are shown in Fig. 2, including 1, 2, 4, 8, 19, 12, 20, or any other suitable number of body patch electrodes, both lager and smaller. Further, in some embodiments, the system 100 may include more than one reference electrode 142, including 2, 4, 5, or any other suitable number of reference patch electrodes. The patch electrodes 140 and reference electrode 142 may be used in generating images or models of a body cavity, such as the chambers of the heart, body lumens that provide access to the body cavity, and other features identified to be electrically isolated from the body cavity (e.g., the pulmonary vein). For example, the patch electrodes 140 may be used in pairs to generate electrical fields in different directions and at different frequencies within a body cavity of a patient. The patch electrodes 140 may be controlled by the mapping and guidance system 114 and/or the patch electrode interface 116 to generate the electrical fields. Mapping data is obtained by the electrodes of the EP catheter 120 by detecting distortions in the electrical fields. The mapping data may then be used to generate the image or model of the body volume, such as a cavity of the heart. The mapping and guidance system 114 may then output the generated map of the cavity to a display. Further, the mapping and guidance system 114 may be configured to determine a location of the EP catheter within the body cavity and output a visualization to the display that indicates a position of the EP catheter 120 within the map. For example, based on the mapping data acquired by the electrodes of the EP catheter, the mapping and guidance system 114 may be configured to determine the position of the electrodes of the EP catheter, and output a visualization indicating the position of each of the electrodes on the map (*see* Fig. 5, indicator 312). Further, based on the determined position of the electrodes of the EP catheter, the mapping and guidance system 114 may be configured to determine or estimate a location of the cryoballoon 132 within the body cavity. For example, in some embodiments, the EP catheter 120 is first advanced to a region of interest, such as the ostium of a pulmonary vein, and anchored into place. The cryoballoon catheter 130 may then be advanced over the EP catheter to the region of interest. If the distance and position of the cryoballoon 132 relative to the electrodes of the EP catheter 120 are fixed and known, the mapping and guidance system 114 may be configured to identify or infer a location of the cryoballoon 132 by a translation of the coordinates of the EP catheter 120. For example, the distance between the cryoballoon 132 and the electrodes of the EP catheter 120 may be added/subtracted to the coordinates of the EP catheter to identify the location of the cryoballoon 132. Accordingly, the mapping and guidance system 114 may be configured to output a visualization of the position of the cryoballoon 132 with respect to the map of the body cavity. A more detailed explanation of the use of body patch electrodes and catheter electrodes to map body volumes and visualize the locations of EP catheters within the map can be found in, for example, U.S. Patent No. 10,278,616, titled "Systems and Methods for Tracking an Intrabody Catheter," filed May 12, 2015, and U.S. Patent No. 5,983,126, titled "Catheter Location System and Method," filed August 1, 1997, the entireties of which are hereby incorporated by reference.

In the diagram shown in Fig. 2, the EP catheter interface 112, mapping and guidance system 114, and patch electrode interface 116 are illustrated as separate components. However, in some embodiments, the interfaces 112, 116 and the mapping and guidance system 114 may be components of a single console or computing device with a single housing. In other embodiments, the interfaces 112, 116 and the mapping and guidance system 114 may comprise separate hardware components (e.g., with separate housings) communicatively coupled to one another by electrical cables, wireless communication devices, fiber optics, or any other suitable means of communication. Further, in some embodiments, the EP catheter interface 112 may also function as an interface for the cryoballoon catheter 130 to control inflation of the cryoballoon 132, cooling/heating of the cryoballoon 132, etc. In other embodiments, the cryoballoon catheter 130 is controlled by a separate interface or control system, such as a console.

The mapping and guidance system 114 is coupled to a display device 118, which may be configured to provide visualizations of a cryoablation procedure to a physician. For example, the mapping and guidance system 114 may be configured to generate EP images of the body cavity, visualizations of the propagation of EP waves across the tissue of the body cavity, indications of occlusion by the cryoballoon at an ablation site, or any other suitable visualization. These visualizations may then be output by the mapping and guidance system 114 to the display device 118.

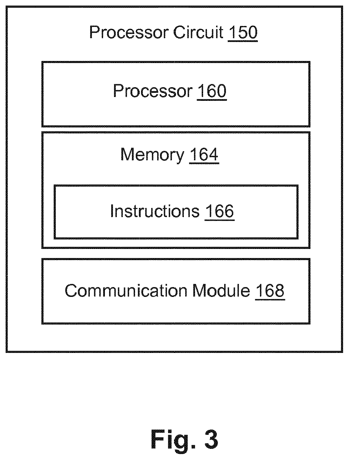

Fig. 3 is a schematic diagram of a processor circuit 150, according to embodiments of the present disclosure. The processor circuit 150 may be implemented in the mapping and guidance system 114, the EP catheter interface 112, the patch electrode interface 116, and/or the display device 118. The processor circuit 150 can carry out one or more steps described herein. As shown, the processor circuit 150 may include a processor 160, a memory 164, and a communication module 168. These elements may be in direct or indirect communication with each other, for example via one or more buses.

The processor 160 may include a central processing unit (CPU), a digital signal processor (DSP), an ASIC, a controller, an FPGA, another hardware device, a firmware device, or any combination thereof configured to perform the operations described herein. The processor 160 may also be implemented as a combination of computing devices, e.g., a combination of a DSP and a microprocessor, a plurality of microprocessors, one or more microprocessors in conjunction with a DSP core, or any other such configuration.

The memory 164 may include a cache memory (e.g., a cache memory of the processor 160), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and nonvolatile memory, or a combination of different types of memory. In an embodiment, the memory 164 includes a non-transitory computer-readable medium. The non-transitory computer-readable medium may store instructions. For example, the memory 164, or non-transitory computer-readable medium may have program code recorded thereon, the program code including instructions for causing the processor circuit 150, or one or more components of the processor circuit 150, to perform the operations described herein. For example, the processor circuit 150 can execute operations of the methods 200, 500, 700. Instructions 166 may also be referred to as code or program code. The terms "instructions" and "code" should be interpreted broadly to include any type of computer-readable statement(s). For example, the terms "instructions" and "code" may refer to one or more programs, routines, subroutines, functions, procedures, etc. "Instructions" and "code" may include a single computer-readable statement or many computer-readable statements. The memory 164, with the code recorded thereon, may be referred to as a computer program product.

The communication module 168 can include any electronic circuitry and/or logic circuitry to facilitate direct or indirect communication of data between the processor circuit 150, the mapping and guidance system 114, the EP catheter 120, the cryoballoon catheter 130, and/or the display 118. In that regard, the communication module 168 can be an input/output (I/O) device. In some instances, the communication module 168 facilitates direct or indirect communication between various elements of the processor circuit 150 and/or the system 100 (Fig. 2). In some embodiments, the processor circuit 150 may further comprise an electrical signal generator and/or an electrical signal measurer configured to control the electrodes of the EP catheter to emit and/or detect electrical signals, including voltages, impedances, and currents.

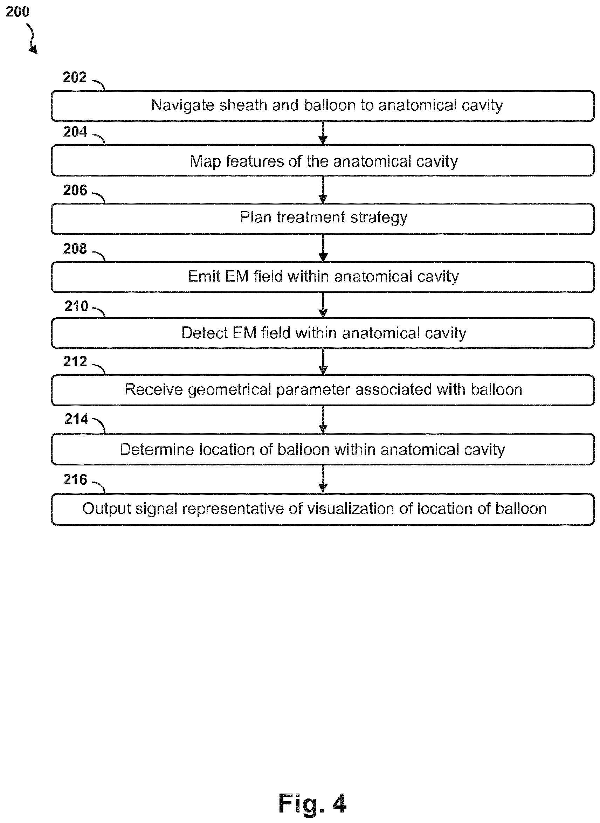

Fig. 4 is a flowchart illustrating a method 200 for performing a balloon therapy procedure, such as balloon ablation or balloon angioplasty, according to some embodiments of the present disclosure. It will be understood that some or all of the steps of the method 200 may be performed using one or more components of the system 100 shown in Fig. 2, including the EP catheter 120, the cryoballoon catheter 130, the mapping and guidance system 114, and/or the patch electrodes 140, 142. In step 202, an EP catheter is navigated to an anatomical cavity, such as a chamber of the heart or a diseased blood vessel. In some embodiments, navigating the EP catheter to the anatomical cavity is performed using EP imaging techniques to image the full anatomical cavity. For example, in some embodiments, a guidewire with electrodes, a sheath with electrodes, and/or the EP catheter is inserted into an entry site, such as the femoral vein in the patient's leg and navigated to the anatomical cavity (e.g., left atrium) while the access route is imaged to guide the placement of the guidewire, sheath, or catheter at the right atrium. In some embodiments, the EP catheter is introduced into the left atrium using a transseptal procedure. In some embodiments, the transseptal procedure may involve the use of a transseptal needle or RF needle to penetrate the transseptal wall to introduce the catheter or sheath into the left atrium.

In step 204, the anatomical cavity is mapped or imaged by moving the EP catheter within the anatomical cavity. For example, for a balloon angioplasty procedure, the vessel walls may be imaged by a guidewire carrying two or more electrodes along a length of the vessel, including a diseased or stenosed portion of the vessel. For balloon ablation, an atrial wall, pulmonary vein (PV) ostia, left atrial appendage (LAA), LAA ridge, mitral valve, or other features of the left atrium may be imaged using the EP catheter 120. Anatomical variations can be detected without the use of contrast media. Accordingly, some or all of the anatomical landmarks relevant to performing the ablation procedure can be mapped using the EP catheter and output to a display to guide preplanning of the ablation process and deployment of the balloon. In some embodiments, the left atrium, right atrium, coronary sinus ostium, septal wall, fossa ovalis, or other features may be imaged using the EP catheter 120.

In some embodiments, mapping of the anatomical cavity is performed in step 204 using the electrodes of the EP catheter to detect distortions in electrical fields generated by external patch electrodes placed on the body of a patient. The distortions in the electrical fields may be caused by the shape and structure of the tissue, and thus the detected distortions can be used to generate a map of the left atrium. In some embodiments, each electrode is configured to emit an electrical signal at a particular frequency. Further, all of the electrodes may be configured to detect the electrical signals emitted by the other electrodes at the other frequencies, in addition to the electrical signal emitted by the electrode. For example, a first electrode may be configured to emit a first electrical signal at a first frequency, and a second electrode may be configured to emit a second electrical signal at a second frequency. In an exemplary embodiment, the first electrode is used to detect the first electrical signal at the first frequency, and the second electrical signal is used to detect the second electrical signal at the second frequency. However, in other embodiments, the first electrode may detect the second electrical signal at the second frequency in addition to its own first electrical signal at the first frequency. Similarly, the second electrode may detect the first electrical signal at the first frequency in addition to its own second electrical signal at the second frequency.

In some embodiments, mapping the anatomical cavity comprises guiding an EP catheter to a plurality of 3D locations, or voxels, within the anatomical cavity, and obtaining voltage measurements at each location with the plurality of electrodes to create a voltage field map, which can then be used by the processor circuit to compute geometrical measurements (e.g., distance) associated with the tissue structures within the anatomical cavity. Further, in some aspects, the processor circuit may generate, based on the voltage measurements, a conductance map showing a conductance field of the anatomical cavity.

With reference to step 204 of the method 200, Fig. 5 shows a user interface 300 that includes three-dimensional images or views of the left atrium. For example, a first atrial view 310 is a perspective three-dimensional view of the internal structure of the left atrium 20 and pulmonary veins 10. The view 310 includes an EP catheter indicator 312 that shows a location and/or orientation of the electrodes at the distal portion or an EP catheter. In some embodiments, the view 310 can be manipulated (e.g., oriented, re-sized, moved) by a user to provide different views or angles of the left atrium. A second atrial view 320 may comprise a flattened panoramic view of the left atrium 20. For example, the second view 320 may be generated by translating, stretching, distorting, or otherwise modifying the first view 310. The second view 320 shows the ostia of the pulmonary veins in a 3D flattened configuration, which may be advantageous for planning and performing ablation procedures, particularly in order to carry out the full ablation procedure without repeated manipulation and frequent changes in projection of the 3D reconstructed image. The views 310, 320 can be used in step 206 to locate the desired treatment site, and to guide placement of a therapeutic balloon at the treatment sites, and/or otherwise plan the treatment procedure. In some embodiments, one or more locations of the EP catheter are recorded during the mapping procedure described with respect to step 204. For example, an initial location of the EP catheter, or any intermediate location of the EP catheter, may be recorded and displayed on the user interface 300 described above.

In step 208, the processor circuit controls a plurality of electrodes to emit an electromagnetic (EM) field within the anatomical cavity. In some embodiments, the electrodes used to emit the EM field include a plurality of body patch electrodes, a plurality of catheter-mounted electrodes, and/or any other suitable type of electrode. Further, it will be understood that the EM field may include multiple EM fields having different parameters or properties, such as different frequencies. In an exemplary embodiment, the electrodes of the EP catheter shown in Fig. 1 may be used to emit the EM field. In some embodiments, a different plurality of catheter-mounted electrodes may be used, including electrodes of a coronary sinus (CS) catheter. In that regard, in some instances, a CS catheter is inserted into an anatomical structure near the anatomical cavity imaged in step 204 to monitor one or more aspects of a balloon ablation procedure. For example, a cryoballoon ablation procedure may involve a risk of neural damage to nerves near the ablation site, such as a phrenic nerve. The CS catheter may include a plurality of electrodes at a distal end of the CS catheter used to obtain EP data of tissue structures near the phrenic nerve to monitor an extent of the cryoballoon ablation. Accordingly, because the CS catheter may already be placed near the anatomical cavity in which the EP catheter and the balloon are positioned, the electrodes of the CS catheter may also be used to emit the EM field of step 208.

In step 210, the processor circuit controls a plurality of electrodes to detect the EM field emitted in step 208. In some embodiments, the electrodes used to detect the EM field include a plurality of body patch electrodes, a plurality of catheter-mounted electrodes, and/or any other suitable type of electrode. In some embodiments, the same plurality of electrodes is used to detect and emit the EM field in steps 208 and 210. For example, in some embodiments, the electrodes of the EP catheter are used to both emit and detect the electrical field, as explained further below. In some embodiments, a first plurality or set of electrodes is used to emit the EM field, and a different, second plurality or set of electrodes is used to detect the EM field. For example, in some embodiments, a plurality of external body patch electrodes is used to emit one or more EM fields within the anatomical cavity, and a plurality of catheter-mounted electrodes (e.g., EP catheter electrodes and/or CS catheter electrodes) is used to detect the EM fields. In some embodiments, a combination of external body patch electrodes and/or catheter-mounted electrodes is used to emit the EM fields, and the same or a different combination of external body patch electrodes and/or catheter-mounted electrodes is used to detect the EM fields. In some embodiments, the processor circuit is configured to control the electrodes to detect the EM field to determine a position of a catheter within the anatomical cavity. In some embodiments, the processor circuit is configured to control the electrodes to detect the EM field to detect or identify a disturbance or change in the EM field that caused by an inflated balloon, for example. In some embodiments, the processor circuit is configured to control the electrodes to detect the EM field to detect a fault condition in which one or more catheter-mounted electrodes are drawn into a sheath, such as a sheath of a balloon ablation catheter, as described further below.

In step 212, the processor circuit receives one or more geometrical parameters associated with the balloon. In some aspects, step 212 may involve retrieving or recalling the one or more geometrical parameters from a memory of the processor circuit. The geometrical parameters may include or represent a set of assumptions of known geometrical conditions of the balloon, including that the electrodes used to emit and/or detect the EM field are positioned distally of the balloon, that a final location of the inflated balloon is in a plane of a previously-imaged pulmonary vein ostium, a shape of the inflated balloon, that the balloon is a continuation of the catheter sheath, a volume of the inflated balloon, a size of the inflated balloon, a dimension of the inflated balloon, and/or any other suitable geometrical parameter associated with the balloon.

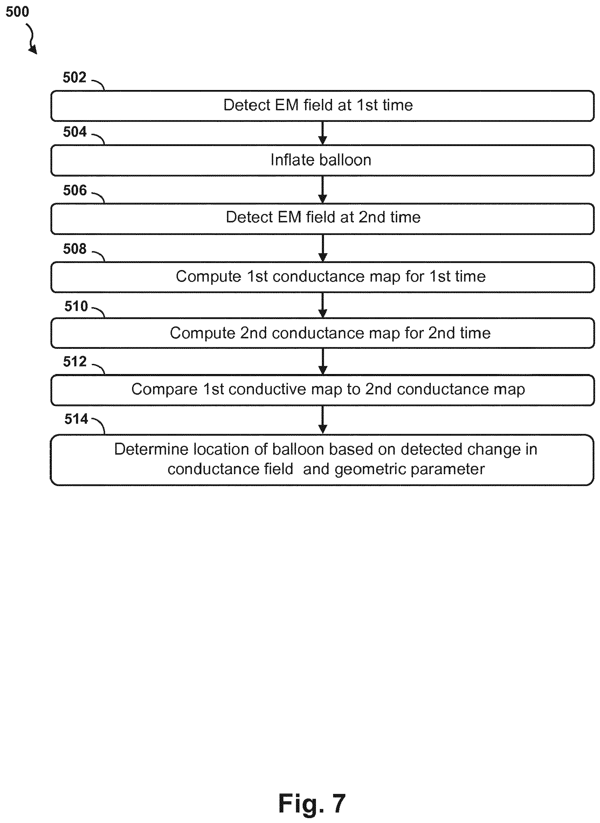

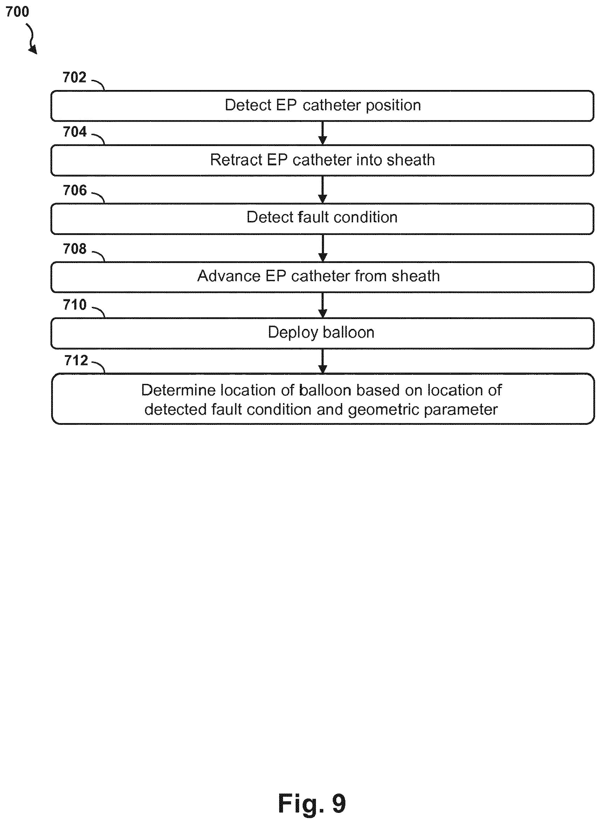

In step 214, the processor circuit determines or computes a location of the balloon within the anatomical cavity based on the geometric parameter and the detected EM field. For example, in some embodiments, detecting the EM field may include detecting or measuring a conductance field, and reconstructing a conductance map. A detected disturbance or change in the conductance field may be used by the processor circuit to estimate or approximate a location of the balloon. Further, the geometrical parameter(s) may be used to improve the accuracy and/or reliability of the detected location. An embodiment using this approach is described below with respect to the method 500 of Fig. 7. In another approach, the processor circuit is configured to determine the location of the balloon based on a fault condition detected during step 210 in which one or more catheter-mounted electrodes are retracted into a sheath or catheter body. In that case, the processor circuit may determine where the fault condition occurred within the anatomical cavity, and infer or compute the location using the location of the fault condition and a geometric parameter associated with the balloon, such as the location of the inflated balloon relative to the distal tip of the sheath. An embodiment using this approach is described below with respect to the method 700 of Fig. 9.

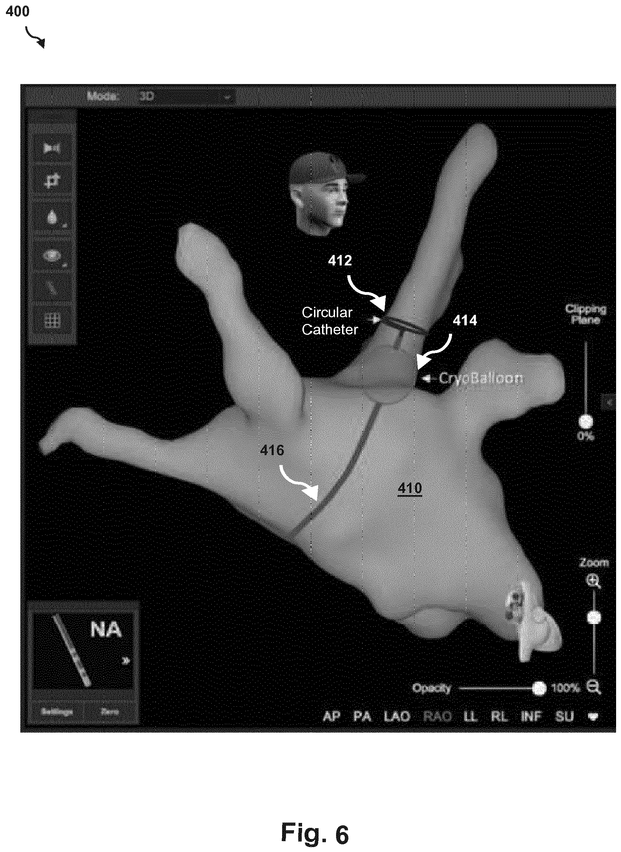

In step 216, the processor circuit outputs a signal representative of a visualization of the location within the anatomical cavity. In some aspects, outputting the visualization may include generating the signal representative of the visualization based on the determined location, and outputting the signal to a display in communication with the processor circuit. The signal may comprise any suitable type of communication, such as an electrical signal (e.g., digital electrical signal) representative of image data. For example, the electrical signal may be in a format suitable for display by a display device (e.g., computer monitor, television screen, mobile computing device display, etc.). In some embodiments, step 216 includes outputting a view or map of the anatomical cavity as performed in step 204, and including the visualization of the location and/or shape of the inflated balloon with respect to the anatomical map of the cavity. For example, the visualization may be overlaid on an anatomical map generated as described above with respect to Fig. 2. In that regard, Fig. 6 depicts a graphical user interface 400 in which the visualization of the location, shape, and size of the balloon 414 is shown within the map of the anatomical cavity. In particular, the map 410 of Fig. 6 is a map of the left atrium, and an indicator of the inflated balloon 414 positioned within the PV ostium. The map 410 may be generated using a plurality of electrodes mounted on a circular catheter or guidewire to detect distortions in electrical fields caused by the tissues of the anatomical structures as described above. The visualization includes an indicator of the inflated balloon 414, an indicator of the balloon catheter 416, and an indicator of the EP electrodes 412. The location of the EP catheter may be determined by detecting changes in an inhomogeneous electrical field created by the atrial wall and other tissues, as described above, while the visualizations of the inflated balloon 414 and/or the catheter bodies are based at least partially on the geometric parameters as in step 214. In an exemplary embodiment, the graphical user interface 400 provides a live, or real-time view of the inflated balloon, such that a current location of the balloon is shown and updated in real time (in accordance with hardware and processing limitations) as the balloon moves around the anatomical cavity.

In some instances, a shape of the visualization 414 of the inflated balloon may be updated in the graphical user interface to represent changes to the shape of the balloon caused by the tissue structures during the treatment procedure. For example, in some embodiments, when the processor circuit determines that the inflated balloon is in contact with and pressing against the PV ostium, the processor circuit may modify the visualization 414 to show a distortion to the shape as the balloon is compressed by the ostium. In that regard, one or more portions of the visualization 414 of the balloon, such as the portion of the balloon in contact with the ostium, may appear somewhat flat in comparison with the spherical visualization shown in Fig. 6. Further, in some embodiments, one or more aspects of the visualization 414 of the inflated balloon may be updated or modified by the processor circuit in response to the treatment procedure (e.g., ablation, radiation) commencing. For example, a color, brightness, or other visual aspect may be updated to show that the treatment procedure has begun.

In some embodiments, a therapeutic balloon may comprise a material having very low conductivity. In some instances, the conductivity of the balloon may be orders of magnitude lower than the surrounding blood or myocardium. Accordingly, a location of the therapeutic balloon can be determined by measuring or detecting a change in a conductance field within the cavity by one or more electrodes (e.g., EP catheter electrodes, CS catheter electrodes, body patch electrodes). Fig. 7 is a flow diagram illustrating a method 500 for visualizing a balloon in a guided balloon ablation procedure, according to aspects of the present disclosure. It will be understood that some or all of the steps of the method 500 may be performed using one or more components of the system 100 shown in Fig. 2, including the EP catheter 120, the cryoballoon catheter 130, the mapping and guidance system 114, and/or the patch electrodes 140, 142. Further, other components may also be used, such as a CS catheter carrying a plurality of catheter-mounted electrodes. Further, it will be understood that the method 500 may correspond to one or more steps of the method 200 described with respect to Fig. 4, such as emitting an EM field within an anatomical cavity in step 208, detecting the EM field in step 210, receiving a geometrical parameter in step 212, determining the location of the balloon in step 214, and outputting the visualization of the balloon in step 216.

In step 502, a processor circuit, which may form part of the mapping and guidance system 114, controls a plurality of electrodes to detect an EM field at a first time. The balloon is not yet inflated at the first time. In an exemplary embodiment, detecting the EM field comprises controlling a plurality of catheter-mounted electrodes (e.g., of an EP catheter, CS catheter, etc.) to obtain electrical measurements, such as voltage measurements, impedance measurements, conductance measurements, etc. For example, an EP catheter may be advanced into the pulmonary vein near the ostium to detect the EM field. As described further below, the positioning of the EP catheter relative to the anatomical features of the patient may be used as a geometrical parameter to identify the location of the balloon. In some embodiments, the same electrodes that are controlled to detect the EM field at the first time are the same electrodes that are used to emit the EM field at the first time. For example, a plurality of catheter-mounted electrodes of an EP catheter may be controlled according to a natives matrix to emit and detect the EM field.

In step 504, the balloon is inflated after the plurality of electrodes measures the EM field in step 502. In some embodiments, the processor circuit is configured to control the inflation or deployment of the ablation balloon and/or the injection of the coolant. In some embodiments, the processor circuit controls the inflation or deployment of the balloon based on a user input received using a user interface device. In some embodiments, a separate control system or processor circuit is used to control the deployment of the balloon. In some embodiments, a separate balloon control system transmits to the processor circuit of the guidance system a signal indicating that the balloon has been inflated. In some embodiments, the balloon is inflated manually by an operator or physician.

In step 506, controls the plurality of electrodes to detect the EM field at a second time, after the first time and after inflating the balloon. In some embodiments, the same electrodes that are controlled to detect the EM field at the second time are the same electrodes that are used to emit the EM field at the second time. For example, a plurality of catheter-mounted electrodes of an EP catheter may be controlled according to a natives matrix to emit and detect the EM field. As described above, in some embodiments, step 506 comprises obtaining voltage measurements using the plurality of electrodes. Because the balloon comprises a nonconductive or dielectric material, the presence of the inflated balloon within the EM field may cause a disturbance or modification to the EM field. Thus, the EM measurements obtained by the electrodes during steps 502 and 506 can be used to identify differences in the EM field (e.g., a conductance field), as further described below.

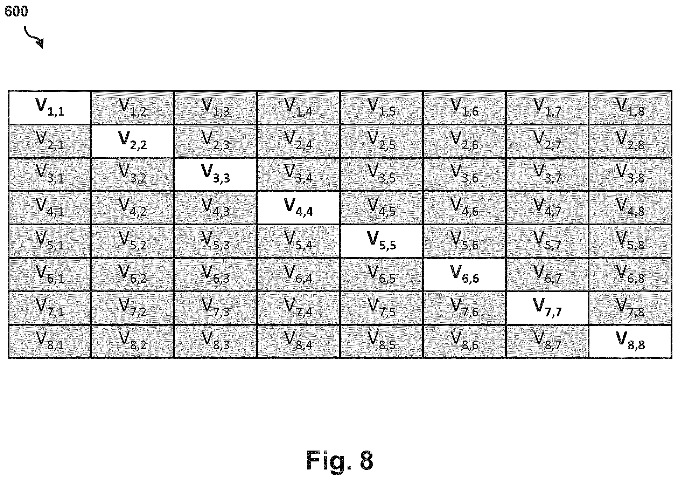

Fig. 8 is a graphical view of a natives matrix 600 representing the signals analyzed by the processor circuit to detect the presence of an inflated balloon, according to one embodiment of the present disclosure. In one aspect, the natives matrix defines combinations of a receiving electrode of a plurality of electrodes and an emitting frequency used to measure an EM field. The voltages of each block are defined as Vij, where i represents which electrode is receiving, and j represents the emitting electrode, or the frequency at which the i^{th} electrode is measuring the signal. Thus, V_{3,5}, for example, is the voltage measured by electrode number 3 at the frequency of electrode number 5. In the table of Fig. 8, the voltages, or electrical signals, that are used to detect the presence of the inflated balloon are not shaded, and the voltages that are not used are shaded. Thus, the voltages that are used comprise a diagonal of the matrix 600, which are the electrical signals that are generated and detected using the same electrodes. For example, the signal V_{1,1}, which is the signal generated and received by the first electrode, is used to detect the presence or location of the inflated balloon, while V_{1,2}, may not be used. However, in other embodiments, any suitable combination of voltages is used. For example, some or all of the non-native voltages are used to detect the balloon. For example, in some embodiments, a mirror-image diagonal of voltages is used in addition to the diagonal in the matrix 600 of Fig. 8. Further, in some embodiments, current, conductance, and/or electrical impedance are used instead of, or in addition to voltage. For example, in some embodiments, the system may be configured to compare impedance measurements to detect the location of the balloon using a given electrode. Further, although the matrix 600 is an 8-by-8 matrix corresponding to an eight-electrode EP catheter, the matrix 600 of electrical signals may include any suitable number of signals, both larger or smaller. For example, as described above, in some embodiments, 2, 4, 6, 8, 10, 12, 15, 20, 30, 60, or any other suitable number of electrodes may be used to detect the presence or location of the balloon. Accordingly, in some embodiments, the matrix 600 may include a 2-by-2 matrix, a 4-by-4 matrix, a 10-by-10 matrix, a 20-by-20 matrix, a 60-by-60 matrix, or any other suitable matrix.

Referring again to Fig. 7, in step 508, the processor circuit computes or generates a first conductance map based on the first EM detected at the first time. In step 510, the processor circuit computes or generates a second conductance map based on the second EM detected at the second time. In some embodiments, the conductance maps are computed based on voltage measurements obtained by catheter-mounted electrodes (e.g., EP catheter, CS catheter). In step 512, the processor circuit compares the first conductance map to the second conductance map to determine a change in a conductance field of the anatomical cavity between the first time and the second time, which represents the changes to the conductance field caused by the presence of the inflated balloon. In some embodiments, step 512 includes subtracting one conductance map from the other conductance map. For example, the first conductance map may be subtracted or deducted from the second conductance map to determine the change in the electric field. In that regard, the conductance map may comprise a matrix of conductance values for a plurality of voxels in a conductance field. Subtracting one conductance field from the other may comprise subtracting values from one conductance field from the spatially-corresponding values from the other conductance field.

In step 514, the processor circuit determines the location of the inflated balloon based on the detected change in the conductance field and the geometric parameter. In some embodiments, the detected change in the conductance field provides an estimate or approximation for the location of the balloon, and the geometric parameter (e.g., distal position of EP catheter relative to the inflated balloon, shape of the inflated balloon, size of the inflated balloon, balloon mechanics, final pose of the balloon relative to the tissue structures of the anatomical cavity, etc.) are used to improve the precision and/or accuracy of the estimated location. In other embodiments, the detected change in the conductance field and the geometric parameter are used as inputs to a relationship to compute the location of the inflated balloon. Once the location of the balloon has been determined, the location of the balloon can be visualized on a display to guide placement of the ablation balloon at the ablation site. As described above, in an exemplary embodiment, the processor circuit is configured to update the visualization of the location of the balloon in real time to provide a live view of the balloon within the anatomical cavity.

According to another embodiment of the present disclosure, a fault condition detection scheme can be used, in addition to one or more geometrical parameters associated with the balloon, to detect the location of the balloon. Fig. 9 is a flow diagram illustrating a method 700 for determining and visualizing a location of an ablation balloon within an anatomical cavity, according to embodiments of the present disclosure. It will be understood that some or all of the steps of the method 700 may be performed using one or more components of the system 100 shown in Fig. 2, including the EP catheter 120, the cryoballoon catheter 130, the mapping and guidance system 114, and/or the patch electrodes 140, 142. Further, other components may also be used, such as a CS catheter carrying a plurality of catheter-mounted electrodes. Further, it will be understood that the method 700 may correspond to one or more steps of the method 200 described with respect to Fig. 4, such as emitting an EM field within an anatomical cavity in step 208, detecting the EM field in step 210, receiving a geometrical parameter in step 212, determining the location of the balloon in step 214, and outputting the visualization of the balloon in step 216.

In step 702, the processor circuit controls a plurality of catheter-mounted electrodes to detect a position of the electrodes relative to the features of the anatomical cavity. In an exemplary embodiment, the processor circuit controls an EP catheter comprising a plurality of electrodes at a distal end of the EP catheter. The EP catheter is positioned at least partially within a lumen of a balloon ablation catheter or sheath, as shown in Fig. 1, for example. Further, the distal portion of the EP catheter having the catheter-mounted electrodes is positioned at a predetermined location near the desired ablation site (e.g., within PV or PV ostium). The processor circuit detects distortions in EM fields generated by external patch electrodes placed on the body of a patient. The distortions in the EM fields may be caused by the shape and structure of the tissue, and thus the detected distortions in the EM fields can be used to generate a map of the anatomical cavity and/or to determine a position of the EP catheter relative to the tissue structures of the anatomical cavity. In some embodiments, the processor circuit is configured to obtain a continuous stream of position data based on voltage measurements obtained by the catheter-mounted electrodes. In that regard, the processor circuit may be configured to detect several EP catheter positions each second, in some embodiments. Further, in some embodiments, a different plurality of electrodes may be used to detect the position in step 702, such as a plurality of CS catheter electrodes.

In step 704, the EP catheter is retracted in a sheath (e.g., sheath 136, Fig. 1), such as the sheath of the balloon catheter. In some embodiments, the EP catheter is manually retracted by the physician. In other embodiments, the processor circuit controls an actuator apparatus or interface device to retract the EP catheter into the sheath. As the EP catheter is retracted into the sheath, the electrodes continue obtaining electrical data or voltage signals while the electrodes of the EP catheter become covered or occluded by the sheath. This results in detectable changes to the voltage measurements obtained by the EP catheter electrodes. In step 706, the processor circuit detects, based on the changes in the electrical measurements, a fault condition for each of the electrodes, as well as the location of the EP catheter when the fault condition occurred. For example, the processor circuit may employ an outlier detection scheme to detect the fault condition. In some embodiments, the fault condition is detected only when all electrodes are retracted into the sheath. In some embodiments, the processor circuit is configured to output a graphical representation of the faulted electrodes to the display to indicate to the physician how many and which of the electrodes have been retracted into the sheath. In step 708, the EP catheter is then advanced out of the sheath such that the detected fault condition is no longer applicable to the electrodes at the distal end of the EP catheter.

In step 710, the balloon is deployed and advanced out of the sheath to the desired treatment location (e.g., PV ostium). In step 712, the processor circuit determines the location of the balloon based on the location associated with the detected fault condition and the geometric parameter associated with the balloon. With reference to Fig. 1, the processor circuit may be able to determine the location of the balloon 132 based on known geometric relationships between the different components. For example, it may be assumed that the location of the deployed balloon 132 will be between the location recorded when the fault condition was detected (e.g., tip of the sheath 136), and the location of the EP catheter electrodes 124 within the PV ostium 10. Further, the volumetric location of the inflated balloon may be determined based on a measured angle trajectory and a plane of the PV ostium previously imaged by the EP catheter.

In some embodiments, the processor circuit is configured to generate and output the visualization of the volumetric location of the balloon immediately or soon after the EP catheter is advanced back out of the sheath. In some embodiments, once the EP catheter is advanced back out of the sheath to the previous location, the EP catheter and the inflated balloon are advanced together so as to maintain the relative longitudinal positioning between the EP catheter electrodes and the inflated balloon. In other embodiments, the balloon may be advanced independently of the EP catheter to the ablation site.

The embodiments of the present disclosure provide for image-guided balloon therapy by detecting and visualizing an inflated balloon, such as a cryoballoon, angioplasty balloon, or scoring balloon, within an anatomical cavity of a patient. However, it will be understood that the embodiments described above are exemplary and are not intended to limit the scope of the present disclosure. In that regard, in some embodiments, the approaches of the methods 200, 500, 700 described above may be to detect and/or visualize other types of therapeutic and/or diagnostic devices to guide other types of therapies, such as balloon angioplasty, laser balloon therapy, balloon breast brachytherapy, drug delivery using drug-coated balloons, neuro-radiology, etc.

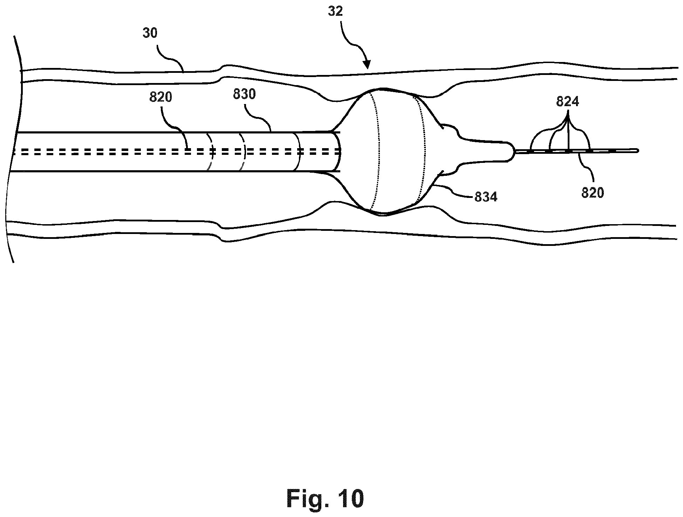

For example, with reference to Fig. 10, a guidewire 820 having a plurality of electrodes 824 at a distal end of the guidewire 820 can be advanced into a stenosed section 32 of a blood vessel 30 to obtain EP data and/or anatomical mapping data of the vessel 30. Accordingly, an anatomical map of the vessel 30 may be generated and displayed. It will be understood that the anatomical map of the vessel 30 may appear similar to the maps generated for other parts of the anatomy, such as the map of the left atrium and pulmonary veins shown in Fig. 6. In that regard, the anatomical map of the vessel 30 may show a cross-sectional view, a partially-transparent view, a volumetric view, or any other suitable view. In some embodiments, the processor circuit is further configured to visualize a location of the guidewire 820 within the map of the vessel 30. Next, an angioplasty balloon catheter 830 is advanced over the guidewire 820 to the stenosed region 32. The angioplasty balloon 834 is then inflated to apply expand or dilate the stenosed region 32. When the balloon 834 is inflated, the electrodes 824 of the guidewire 820 can be used to detect changes in the conductance field caused by the presence of the inflated balloon 834, as described with respect to the method 500. Alternatively, the guidewire 820 can be temporarily withdrawn into the sheath of the catheter 830 while its location is recorded, and then advanced out of the catheter 830 to determine the relative location of the balloon 834, as described with respect to the method 700. A visualization of the location of the balloon 834 can then be output to the display to guide placement of the balloon 834 at the stenosed segment 32.

In still other embodiments, a drug-coated balloon, a radiation balloon, a scoring balloon, or any other suitable treatment balloon is advanced over a guidewire or catheter to a treatment site. Using one or more of the approaches described above, the therapy balloon can be spatially localized and visualized within the vessel relative to the treatment site (e.g., stenosis, lesion, tumor, etc.) to ensure proper placement and deployment of the therapy balloon. This approach may be particularly advantageous in neuro radiology and/or vascular balloon therapy procedures in which the therapeutic balloons are too small to carry electrodes for visualization.

Further, in some embodiments, once a therapeutic device, such as an ablation balloon, has been positioned at the ablation site using the techniques described above, the processor circuit may perform other guidance techniques, such as leak detection, to verify that the balloon fully occludes the ablation site (e.g., PV ostium). For example, a balloon ablation guidance system may use one or more of the techniques described in U.S. Publication No. 2018/0125575, titled "LESION ASSESSMENT BY DIELECTRIC PROPERTY ANALYSIS," filed May 11, 2016, U.S. Patent Application Publication No. 2018/0116751, titled "CONTACT QUALITY ASSESSMENT BY DIELECTRIC PROPERTY ANALYSIS," filed May 11, 2016, and/or European Patent Application 19184189.9, titled "ELECTROPHYSIOLOGICAL GUIDANCE OF BALLOON ABLATION AND ASSOCIATED DEVICES, SYSTEMS, AND METHODS," filed July 3, 2019, the entireties of which are hereby incorporated by reference. Further, it will be understood that one or more techniques described above, such as the techniques of the methods 500 and 700, may be combined to detect and visualize a location of an ablation balloon to guide an ablation procedure.

It will be understood that one or more of the steps of the methods 200, 500, 700, such as generating the map of a body cavity, detecting the location of the inflated balloon, and outputting visualizations to a display indicating the detected location of the balloon, can be performed by one or more components of an EP-guided ablation system, such as a processor circuit of a mapping and guidance system, an EP catheter, a cryoballoon catheter, an RF ablation balloon catheter, external body patch electrodes, or any other suitable component of the system. For example, the described ablation procedures may be carried out by the system 100 described with respect to Fig. 2, which may include the processor circuit 150 described with respect to Fig. 3. In some embodiments, the processor circuit can use hardware, software or a combination of the two to perform the analyses and operations described above. For example, the result of the signal processing steps of the methods 200, 500, 700 may be processed by the processor circuit executing a software to make determinations about the locations of the electrode in the 3D image, etc.

It will also be understood that the embodiments described above are exemplary and are not intended to limit the scope of the disclosure to a given clinical application. For example, as mentioned above, the devices, systems, and techniques described above can be used in a variety of balloon ablation applications that involve occlusion of a body cavity or body lumen. For example, in some embodiments, the techniques described above can be used to guide a cryoablation procedure using a cryocatheter comprising a cryoballoon as described above. In other aspects, the techniques described above can be used to guide an RF ablation procedure in which a plurality of RF ablation electrodes positioned on the surface of an inflatable balloon are used to create an electrically-isolating lesion in cardiac tissue. For example, the HELIOSTAR RF balloon catheter, manufactured by Biosense Webster, Inc., includes 10 ablation electrodes positioned on an external surface of the inflatable balloon, and 10 electrodes on a circular mapping catheter positioned distally of the balloon and configured to be positioned inside the pulmonary vein.

Further, while the ablation procedures are described with respect to the heart and associated anatomy, it will be understood that the same methods and systems can be used to guide ablation procedures in other body volumes, including other regions of interest in the heart, or other body cavities and/or lumens. For example, in some embodiments, the EP guided ablation procedures described herein can be used to guide treatment procedures in any number of anatomical locations and tissue types, including without limitation, organs including the liver, heart, kidneys, gall bladder, pancreas, lungs; ducts; intestines; nervous system structures including the brain, dural sac, spinal cord and peripheral nerves; the urinary tract; as well as valves within the blood, chambers or other parts of the heart, and/or other systems of the body. The anatomy may be a blood vessel, as an artery or a vein of a patient's vascular system, including cardiac vasculature, peripheral vasculature, neural vasculature, renal vasculature, and/or any other suitable lumen inside the body. In addition to natural structures, the approaches described herein may be used to examine man-made structures such as, but without limitation, heart valves, stents, shunts, filters and other devices. in the kidneys, lungs, or any other suitable body volume. Further, the balloon detection and visualization techniques described above can be employed in various applications to determine the location of a balloon. For example, the procedures described above can be used for intravascular balloon-based stenosis treatment, or any other suitable application.

Persons skilled in the art will recognize that the apparatus, systems, and methods described above can be modified in various ways. Accordingly, persons of ordinary skill in the art will appreciate that the embodiments encompassed by the present disclosure are not limited to the particular exemplary embodiments described above. In that regard, although illustrative embodiments have been shown and described, a wide range of modification, change, and substitution is contemplated in the foregoing disclosure. It is understood that such variations may be made to the foregoing without departing from the scope of the present disclosure. Accordingly, it is appropriate that the appended claims be construed broadly and in a manner consistent with the present disclosure.

## Claims

1. An apparatus for guiding balloon therapy within an anatomical cavity, comprising:
a processor circuit configured for communication with a plurality of electrodes, wherein the processor circuit is configured to:
control the plurality of electrodes to detect an electromagnetic field within the anatomical cavity;
receive a geometrical parameter associated with a balloon configured to provide the balloon therapy within the anatomical cavity;
determine, based on the detected electromagnetic field and the geometrical parameter associated with the balloon, a location of the balloon within the anatomical cavity; and
output a signal representative of a visualization of the location of the balloon within the anatomical cavity.

2. The apparatus of claim 1, wherein the processor circuit is configured to:
control the plurality of electrodes to detect the electromagnetic field at a first time before the balloon is inflated and a second time after the balloon is inflated,
compute, based on the electromagnetic field detected at the first time, a first conductance map of the anatomical cavity corresponding to the first time;
compute, based on the electromagnetic field detected at the second time, a second conductance map of the anatomical cavity corresponding to the second time; compare the first conductance map and the second conductance map to determine a change in a conductance field of the anatomical cavity between the first time and the second time; and
determine, based on the geometrical parameter and the determined change in the conductance field, the location of the balloon within the anatomical cavity.

3. The apparatus of claim 2, wherein the processor circuit is configured to control a plurality of catheter-mounted electrodes to detect the electromagnetic field at the first time and the second time.

4. The apparatus of claim 3, wherein the processor circuit is configured to control the plurality of catheter-mounted electrodes to emit the electromagnetic field at the first time and the second time.

5. The apparatus of claim 4, wherein the processor circuit is configured to control the plurality of catheter-mounted electrodes to detect the electromagnetic field based on combinations of a receiving electrode of the plurality of catheter-mounted electrodes and an emitting frequency.

6. The apparatus of claim 3, wherein the processor circuit is configured to control a plurality of external electrodes to emit the electromagnetic field at the first time and the second time.

7. The apparatus of claim 1, wherein the processor circuit is configured to:
control a plurality of catheter-mounted electrodes to detect the electromagnetic field within the anatomical cavity;
control the plurality of catheter-mounted electrodes to detect a fault condition in which each catheter-mounted electrode of the plurality of catheter-mounted electrodes is occluded; and
determine the location of the balloon within the anatomical cavity based on the detected fault condition and the geometric parameter associated with the balloon.

8. The apparatus of claim 1, wherein the geometrical parameter comprises one or more of:
a relative positioning of the balloon relative to the plurality of electrodes;
a shape of the balloon;
a boundary condition of the balloon within the anatomical cavity; or
a geometric dimension of the balloon when inflated.

9. The apparatus of claim 1, wherein the visualization of the location of the balloon comprises a graphical representation of a shape of the balloon when inflated, within the anatomical cavity, and wherein the processor circuit is configured to update the graphical representation of the shape of the balloon positioned relative to the anatomical cavity, in real time, based on the detected electromagnetic field.

10. The apparatus of claim 1, wherein the processor circuit is configured to output the signal representative of the visualization of the location of the balloon to a display in communication with the processor circuit.

11. A system for guiding balloon therapy within an anatomical cavity, comprising:
the apparatus as in any of claims 1-10; and
an electrophysiology catheter comprising an elongate tip member configured to be positioned distally of the balloon, wherein the plurality of electrodes is positioned on the elongate tip member.

12. A method for guiding balloon therapy within an anatomical cavity, comprising:
controlling a plurality of electrodes to detect an electromagnetic field within the anatomical cavity;
receiving a geometrical parameter associated with a balloon configured to provide the balloon therapy within the anatomical cavity;
determining, based on the detected electromagnetic field and the geometrical parameter associated with the balloon, a location of the balloon within the anatomical cavity; and
outputting a signal representative of a visualization of the location of the balloon within the anatomical cavity.

13. The method of claim 12,
wherein controlling the plurality of electrodes comprises:
controlling the plurality of electrodes to detect the electromagnetic field at a first time before inflating the balloon; and
controlling the plurality of electrodes to detect the electromagnetic field at a second time after inflating the balloon; and
wherein determining the location of the balloon within the anatomical cavity comprises:
computing, based on the electromagnetic field detected at the first time, a first conductance map of the anatomical cavity corresponding to the first time;
computing, based on the electromagnetic field detected at the second time, a second conductance map of the anatomical cavity corresponding to the second time;
comparing the first conductance map and the second conductance map to determine a change in a conductance field of the anatomical cavity between the first time and the second time; and
determining, based on the geometrical parameter and the determined change in the conductance field, the location of the balloon within the anatomical cavity.

14. The method of claim 12,
wherein controlling the plurality of electrodes comprises controlling a plurality of catheter-mounted electrodes to detect the electromagnetic field within the anatomical cavity, and
wherein determining the location of the balloon within the anatomical cavity comprises:
controlling the plurality of catheter-mounted electrodes to detect a fault condition in which each catheter-mounted electrode of the plurality of catheter-mounted electrodes is occluded; and
determining, based on the detected fault condition and the geometric parameter associated with the balloon, the location of the balloon within the anatomical cavity.

15. A computer program product comprising:
a non-transitory computer-readable medium having program code recorded thereon, the program code including:
code for controlling a plurality of electrodes to detect an electromagnetic field within an anatomical cavity;
code for receiving a geometrical parameter associated with a balloon configured to provide therapy within the anatomical cavity;
code for determining, based on the detected electromagnetic field and the geometrical parameter associated with the balloon, a location of the balloon within the anatomical cavity; and
code for outputting a signal representative of a visualization of the location of the balloon within the anatomical cavity.
